# FASCICULE DE BREVET EUROPEEN

(11) **EP 1 121 953 B1**
(45) Date de publication et mention de la délivrance du brevet: **22.10.2003**
(21) Numéro de dépôt: 01400240.6
(22) Date de dépôt: 31.01.2001
(51) Int. Cl.: A61M 16/06

(54) **Appareil d'assistance respiratoire**
Beatmungsvorrichtung
Respiratory assistance apparatus

(30) Priorité: 01.02.2000 FR 0001240
(43) Date de publication de la demande: 08.08.2001
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- EP-A- 0 747 078
- DE-A- 3 707 952
- DE-A- 19 845 415
- US-A- 2 931 356
- US-A- 4 971 051
- US-A- 5 139 510
- US-A- 5 269 296
- US-A- 5 660 174

## Description

La présente invention a pour objet un appareil d'assistance respiratoire, utilisable sur des patients dont la respiration spontanée est absente ou insuffisante.

On connaît déjà des appareils d'assistance respiratoire (voir par ex. US-A-4971051) permettant d'amener, dans les poumons d'un patient, du gaz respiratoire provenant d'une source extérieure, lesdits appareils comportant :
- une chambre pourvue d'une entrée de gaz respiratoire destinée à être reliée à ladite source et d'une sortie de gaz respiratoire destinée à être reliée à une voie respiratoire dudit patient ; et
- un organe d'appui, par exemple un bourrelet de mousse ou une vessie souple gonflable, destiné à prendre appui sur une partie dudit patient pour assurer l'étanchéité entre ladite sortie de gaz respiratoire et l'extérieur.

De tels appareils d'assistance respiratoire connus peuvent, par exemple, prendre la forme :
- d'un masque respiratoire, dont ladite chambre est délimitée par une coque creuse destinée à être appliquée, par son ouverture qui constitue ladite sortie de gaz, sur le visage du patient en enfermant le nez de celui-ci. L'entrée de gaz respiratoire est alors formée par un embout solidaire du fond de ladite coque, tandis que ledit organe d'appui présente la forme d'un bourrelet, en une matière mousse ou sous forme d'une vessie gonflable, suivant le contour de l'ouverture de ladite coque et interposé entre ladite ouverture et le visage du patient ; ou bien encore
- d'un dispositif d'intubation nasal, dont ladite chambre est délimitée par un élément tubulaire destiné à être introduit dans une narine du patient. Dans ce cas, lesdites entrée et sortie de gaz respiratoire sont formées par les extrémités opposées dudit élément tubulaire et ledit organe d'appui est une vessie gonflable se présentant sous la forme d'un ballonnet porté par la paroi externe dudit élément tubulaire et interposé entre celui-ci et la paroi interne de la narine.

Quels que soient les modes de réalisation de ces appareils d'assistance respiratoire connus, ceux-ci présentent l'inconvénient que leur organe d'appui ne peut assurer une étanchéité satisfaisante. En effet, pour des raisons de résistance mécanique, ledit organe d'appui doit présenter une certaine rigidité, qui lui interdit en conséquence d'épouser avec exactitude les irrégularités du relief de la partie dudit patient (visage ou paroi interne de narine par exemple) sur laquelle elle est appliquée. Il en résulte donc des fuites, entraînant de coûteuses pertes de gaz respiratoire. De plus, dans le cas d'un masque, le gaz respiratoire passant entre le bourrelet et le visage du patient par suite des défauts d'étanchéité dudit bourrelet pénètre dans les yeux du patient et est la cause d'irritations oculaires et de conjonctivites.

Pour compenser de tels défauts d'étanchéité, les praticiens sont amenés :
- à accroître la pression d'application dudit organe d'appui sur la partie correspondante du patient. Un tel accroissement de pression d'application est obtenu soit par application avec force du masque sur le visage (à l'aide de sangles élastiques passant derrière la tête par exemple), soit par augmentation de la pression interne du ballonnet du dispositif d'intubation nasal. Cependant, une pression d'application élevée dudit organe d'appui entraîne la formation d'escarres aux endroits où l'appareil respiratoire s'applique sur le patient ; et/ou
- à accroître la pression à laquelle le gaz respiratoire est amené à l'appareil respiratoire. Dans ce cas, il peut en résulter des blessures des muqueuses atteintes par le gaz respiratoire. De plus, dans ce dernier cas, les fuites de gaz subsistent --et sont même augmentées-- avec leurs inconvénients.

L'invention a pour but de remédier à ces inconvénients, et elle concerne un appareil d'assistance respiratoire du type indiqué précédemment, grâce auquel tout risque de fuite de gaz respiratoire, au niveau de l'organe d'appui, peut être évité.

A cet effet, selon l'invention, l'appareil d'assistance respiratoire permettant d'amener, dans les poumons d'un patient, du gaz respiratoire provenant d'une source extérieure, ledit appareil comportant :
- une chambre pourvue d'une entrée de gaz respiratoire destinée à être reliée à ladite source et d'une sortie de gaz respiratoire destinée à être reliée à une voie respiratoire dudit patient ; et
- un organe d'appui, destiné à prendre appui sur une partie dudit patient pour assurer l'étanchéité entre ladite sortie de gaz respiratoire et l'extérieur,
est remarquable :
- en ce qu'il comporte une enveloppe souple gonflable enveloppant ledit organe d'appui ; et
- en ce que la souplesse de ladite enveloppe est plus grande que celle dudit organe d'appui ; et
- en ce que ladite enveloppe souple est, d'un côté dudit organe d'appui alimentée en gaz respiratoire, et, de l'autre côté dudit organe d'appui, en communication avec l'extérieur pour évacuer ledit gaz respiratoire, de sorte que lorsque ledit organe d'appui ne peut pas s'appliquer avec étanchéité contre ladite partie d'appui du patient, l'espace qui en résulte est obturé par ladite enveloppe souple gonflée.

Ceci assure ainsi l'étanchéité d'application aux endroits où cette étanchéité ne peut être obtenue par ledit organe d'appui. De plus, ladite enveloppe souple est alors gonflée par le gaz respiratoire sous pression provenant de ladite chambre et s'évacuant à l'extérieur.

De préférence, ladite enveloppe souple est réalisée dans un film plastique de quelques microns d'épaisseur.

Dans le cas où ledit appareil d'assistance respiratoire présente la forme d'un masque, dont ladite chambre est définie par une coque creuse destinée à être appliquée, par son ouverture qui constitue ladite sortie de gaz, sur le visage d'un patient en enfermant le nez de celui-ci, l'entrée du gaz respiratoire étant prévue dans le fond de ladite coque et ledit organe d'appui présentant la forme d'un bourrelet suivant le contour de l'ouverture de ladite coque et interposé entre ladite ouverture et ledit visage du patient, il est avantageux que :
- ladite enveloppe souple soit un manchon replié autour dudit bourrelet, ledit manchon comportant une partie interne à ladite coque et une partie externe à ladite coque, reliées l'une à l'autre par une partie intermédiaire recourbée autour dudit bourrelet ;
- ladite partie interne de l'enveloppe souple définisse avec la paroi interne de ladite coque un espace interne, par exemple en communication avec ladite chambre ;
- ladite partie externe de l'enveloppe souple définisse avec la paroi externe de ladite coque un espace externe, par exemple en communication avec l'extérieur ; et
- ledit espace interne soit en communication avec ladite chambre, tandis que ledit espace externe est en communication avec l'extérieur.

Un tel manchon peut, soit être rapporté de façon amovible à ladite coque, soit être solidaire de cette dernière. Dans ce dernier cas, il est avantageux que ledit manchon soit solidarisé de la coque au moment de la fabrication du masque d'assistance respiratoire, qui peut alors être du type jetable après usage. Au contraire, dans le premier cas, le manchon peut être rapporté à tout moment à un masque respiratoire initialement fabriqué sans manchon.

Afin d'assurer un gonflage permanent de ladite enveloppe souple, il est préférable de prévoir que la section de passage totale du ou des orifices pratiqués à travers ladite partie interne du manchon soit plus grande que la section de passage totale du ou des orifices pratiqués à travers ladite partie externe dudit manchon.

Dans une variante de réalisation de l'appareil d'assistance respiratoire conforme à la présente invention, dans laquelle ledit appareil comporte un élément tubulaire, dont le volume interne délimite ladite chambre et qui est destiné à être introduit dans une narine du patient, lesdites entrée et sortie de gaz respiratoire étant formées par les extrémités opposées dudit élément tubulaire et ledit organe d'appui se présentant sous la forme d'un ballonnet porté par la paroi externe dudit élément tubulaire et interposé entre celui-ci et la paroi interne de ladite narine, ladite enveloppe souple peut présenter la forme d'un ballon enveloppant ledit ballonnet et porté par ladite paroi externe dudit élément tubulaire et ledit ballon peut être en communication, à son extrémité distale, avec ladite chambre par l'intermédiaire d'au moins un orifice pratiqué à travers la paroi dudit élément tubulaire et, à son extrémité proximale, avec l'extérieur, par l'intermédiaire d'au moins un orifice pratiqué à travers la paroi dudit ballon.

Là encore, et pour la même raison que celle mentionnée ci-dessus, la section de passage totale du ou des orifices pratiqués à travers ladite paroi de l'élément tubulaire est avantageusement plus grande que la section de passage totale du ou des orifices pratiqués à travers la paroi dudit ballon.

Les figures du dessin annexé feront bien comprendre comment l'invention peut être réalisée. Sur ces figures, des références identiques désignent des éléments semblables.

La figure 1 illustre schématiquement, en coupe axiale, un exemple de réalisation de l'appareil d'assistance respiratoire conforme à la présente invention, se présentant sous la forme d'un masque.

La figure 2 illustre schématiquement, également en coupe axiale, une variante de réalisation du masque de la figure 1.

Les figures 3A et 3B illustrent schématiquement, en vues partielles agrandies, le processus selon lequel l'étanchéité est obtenue avec les masques des figures 1 et 2.

La figure 4 illustre schématiquement, en coupe axiale, une variante de réalisation de l'appareil d'assistance respiratoire conforme à la présente invention, se présentant sous la forme d'un tube.

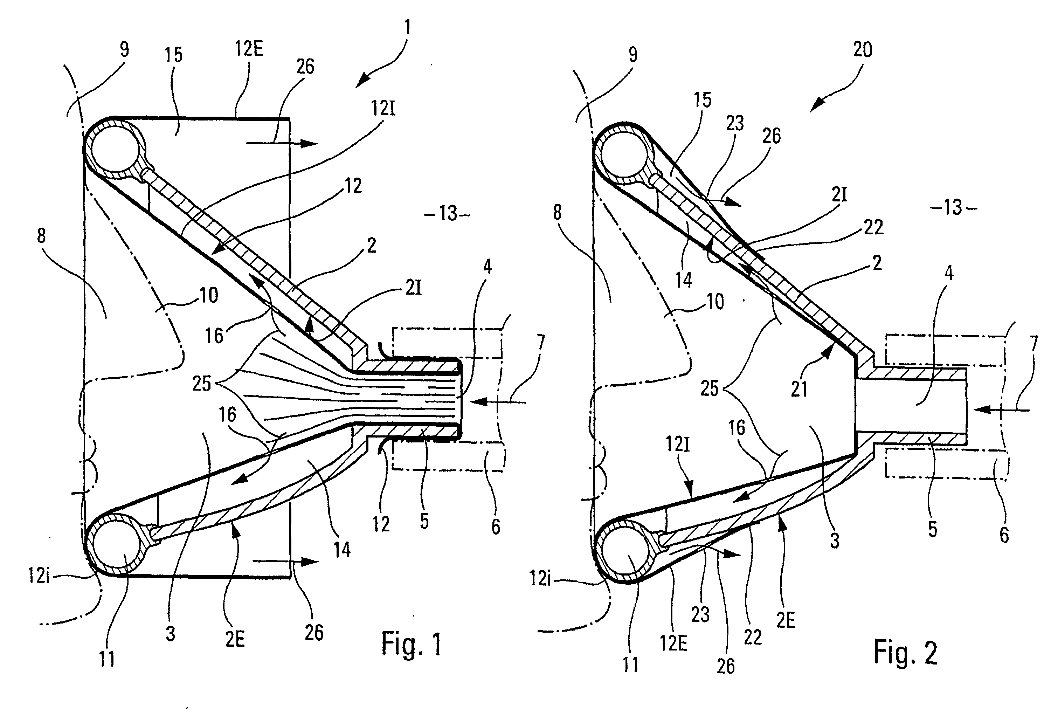

L'appareil d'assistance respiratoire 1, conforme à la présente invention et montré par la figure 1, se présente sous la forme d'un masque respiratoire comportant une coque rigide creuse 2 délimitant une chambre intérieure 3. Au fond de la coque 2 est aménagée une entrée de gaz respiratoire 4, par exemple grâce à un embout tubulaire 5, solidaire de ladite coque, pouvant être reliée à une source de gaz respirable (non représentée), par exemple une bouteille sous pression, par une tubulure appropriée 6. Sur la figure 1, l'arrivée de gaz respirable est symbolisée par la flèche 7.

La chambre intérieure 3 comporte une sortie du gaz respiratoire constituée par l'ouverture 8 de ladite coque rigide 2. Celle-ci est destinée à être appliquée, par son ouverture 8, sur le visage 9 d'un patient (représenté en traits mixtes) en enfermant le nez 10 de celui-ci.

Afin d'assurer l'étanchéité au gaz entre l'ouverture 8 de la coque 2 et le visage 9, l'appareil respiratoire 1 comporte un organe d'appui 11 constitué par une vessie gonflable à paroi mince se présentant sous la forme d'un bourrelet, solidaire de la coque 2 et suivant le contour de l'ouverture 8 de cette dernière, ledit bourrelet 11 étant interposé entre ladite ouverture 8 et le visage 9 du patient lorsque la coque 2 est appliquée contre le visage 9 du patient.

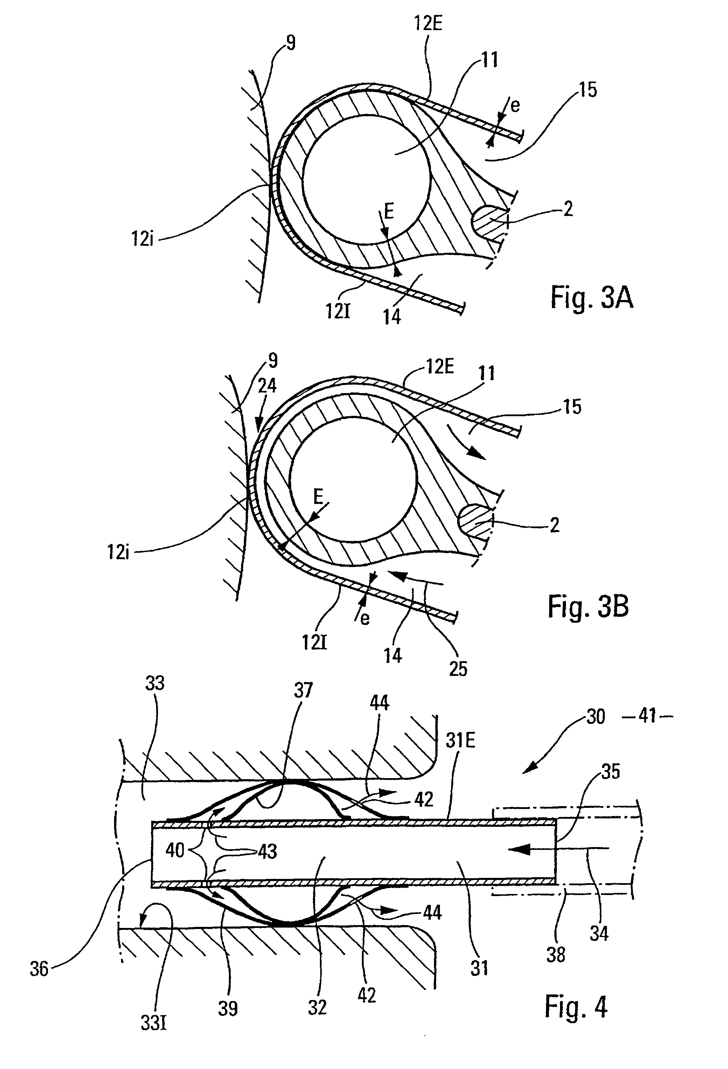

Conformément à la présente invention, l'appareil d'assistance respiratoire 1 comporte de plus une enveloppe souple 12, dont l'épaisseur de paroi e est très inférieure à celle E dudit bourrelet 11 (voir les figures 3A, 3B), de sorte que ladite enveloppe souple 12 peut présenter une bien plus grande souplesse que ce dernier. Par exemple, l'enveloppe souple 12 est réalisée en un film plastique de quelques microns d'épaisseur.

L'enveloppe souple 12 enveloppe le bourrelet 11 et est en communication, d'une part avec la chambre intérieure 3 et, d'autre part, avec l'extérieur 13. A cet effet, l'enveloppe 12 se présente sous la forme d'un manchon, par exemple cylindrique ou conique, replié autour du bourrelet 11 et comporte :
- une partie interne 12I, qui est disposée à l'intérieur de la coque 2, dans la chambre 3 de celle-ci, et qui définit, avec la paroi interne 2I de ladite coque 2, un espace interne 14 ;
- une partie externe 12E, qui est disposée à l'extérieur de la coque 2 et qui définit, avec la paroi externe 2E de cette dernière, un espace externe 15 ; et
- une partie intermédiaire 12i, recourbée autour du bourrelet 11 et reliant lesdites parties interne et externe 12I et 12E.

Dans l'exemple de réalisation représenté sur la figure 1, le manchon 12 est rapporté de façon amovible à la coque 2 et est fixé à celle-ci par exemple par passage de la partie interne 12I à travers l'embout tubulaire 5 et repliement autour de l'extrémité libre de celui-ci. Le manchon 12 peut alors être attaché de façon amovible du fait que son extrémité libre repliée autour dudit embout tubulaire 5 est pincée entre celui-ci et la tubulure 6. De plus, la communication entre l'espace interne 14 et la chambre intérieure 3 est assurée par des orifices 16 pratiqués à travers la partie interne 12I du manchon 12, alors que l'espace externe 15 est ouvert vers l'extérieur 13.

Dans la variante de réalisation 20 de l'appareil d'assistance respiratoire conforme à la présente invention montré par la figure 2, on retrouve les différents éléments 2 à 16 décrits en regard de la figure 1. Cependant, dans l'appareil 20, le manchon 12 est définitivement solidaire de la coque 2 et, par rapport à l'appareil 1, cet appareil 20 présente les différences suivantes :
- la partie interne 12I est collée ou soudée en 21 sur la paroi interne 2I de la coque 2 ;
- la partie externe 12E est collée ou soudée en 22 sur la paroi externe 2E de la coque 2, de sorte que l'espace 15 est refermé et que ladite partie externe 12E doit comporter des orifices 23 pour mettre en communication l'espace 15 avec l'extérieur 13. Dans ce cas, il est avantageux que la section de passage totale du ou des orifices 16 soit plus grande que la section de passage totale du ou des orifices 23.

Lorsque le masque d'assistance respiratoire 1 ou 20 est appliqué sur le visage 9 d'un patient, le bourrelet 11, à cause de sa rigidité relative et des irrégularités du relief de ce visage, ne peut s'appliquer uniformément sur ce dernier de façon rigoureusement étanche. Certes, sur la plus grande partie de son contour, ledit bourrelet 11 s'applique avec étanchéité contre le visage 9, comme cela est illustré sur la figure 3A. Dans ce cas, la partie intermédiaire 12i de l'enveloppe 12 est elle-même pressée entre le bourrelet 11 et le visage 9. En revanche, en certains endroits dudit bourrelet 11, celui-ci est maintenu écarté du visage 9 en laissant subsister un espace 24 avec ce dernier (voir la figure 3B).

Dans ce dernier cas, le gaz respiratoire pénétrant dans la coquille 2 par l'ouverture 4 (flèche 7) et passant dans l'espace interne 14 à travers les orifices 16 (flèches 25) gonfle la partie intermédiaire 12i de l'enveloppe souple 12 qui vient s'appliquer avec étanchéité contre le visage 9 en obturant ledit espace 24. Ce gaz passe alors entre le bourrelet 11 et la partie intermédiaire d'enveloppe 12i et pénètre dans l'espace externe 15, d'où il s'évacue vers l'extérieur 13 (flèches 26), soit par l'ouverture du manchon 12 (figure 1), soit à travers les orifices 23 (figure 2).

On voit qu'ainsi l'enveloppe 12 permet de parfaire l'étanchéité des appareils 1 et 20, aux emplacements où cette étanchéité ne peut être assurée par le bourrelet 11.

Dans la variante de réalisation illustrée schématiquement par la figure 4, l'appareil d'assistance respiratoire 30 conforme à la présente invention se présente sous la forme d'un élément tubulaire 31, dont le volume interne délimite une chambre 32 et qui est destiné à être introduit dans une narine 33 d'un patient. Du gaz respiratoire, provenant d'une source, non représentée mais symbolisée par la flèche 34, est introduit, par une tubulure 38, dans ledit élément tubulaire 31 à travers l'extrémité proximale 35 de ce dernier et est adressé aux poumons dudit patient à travers l'extrémité distale 36 dudit élément tubulaire, débouchant de la narine 33.

L'appareil 30 comporte, de façon connue, un organe d'appui constitué par une vessie gonflable de maintien et d'étanchéité 37, se présentant sous la forme d'un ballonnet apte à se dilater pour venir s'appuyer contre la paroi interne 33I de la narine 33. Le ballonnet 37 est porté par la paroi externe 31E de l'élément tubulaire 31 et est interposé entre ce dernier et ladite paroi interne 33I de la narine 33.

Conformément à la présente invention, le ballonnet 37 est enveloppé par un ballon 39, porté par ladite paroi externe 31E de l'élément tubulaire 31, ledit ballon 39 étant réalisé en un film plastique très mince.

Le ballon 39 est en communication, à son extrémité distale, avec la chambre 32 par l'intermédiaire d'orifices 40 pratiqués à travers la paroi de l'élément tubulaire 31, entre les extrémités distales du ballonnet 37 et du ballon 39. A son extrémité proximale, le ballon 39 est en communication avec l'extérieur 41, par l'intermédiaire d'orifices 42 pratiqués à travers la paroi du ballon 39.

On comprendra aisément que, de façon semblable à ce qui a été expliqué ci-dessus en regard des figures 3A et 3B à propos du manchon 12, le ballon 39 est apte à assurer l'étanchéité entre la sortie 36 de l'élément tubulaire 31 et la paroi interne 33I de la narine 33, aux endroits où le ballonnet 37 présente des défauts d'étanchéité.

## Revendications

1. Appareil d'assistance respiratoire (1, 20, 30) permettant d'amener, dans les poumons d'un patient, du gaz respiratoire provenant d'une source extérieure, ledit appareil comportant :
- une chambre (3, 32) pourvue d'une entrée de gaz respiratoire (4, 35) destinée à être reliée à ladite source et d'une sortie de gaz respiratoire (8, 36) destinée à être reliée à une voie respiratoire (10, 33) dudit patient ; et
- un organe d'appui (11, 37), destiné à prendre appui sur une partie (9, 331) dudit patient pour assurer l'étanchéité entre ladite sortie de gaz respiratoire et l'extérieur,
**caractérisé :**
- **en ce qu'**il comporte une enveloppe souple (12, 39) enveloppant ledit organe d'appui (11, 37) ;
- **en ce que** la souplesse de ladite enveloppe (12, 39) est plus grande que celle dudit organe d'appui (11, 37) ; et
- **en ce que** ladite enveloppe souple est, d'un côté dudit organe d'appui (11, 37), alimentée en gaz respiratoire, et, de l'autre côté dudit organe d'appui, en communication avec l'extérieur (13, 41) pour évacuer ledit gaz respiratoire, de sorte que lorsque ledit organe d'appui (11,37) ne peut pas s'appliquer avec étanchéïté l'espace qui en résulte est obturé par ladite enveloppe souple gonflée (12, 39).

2. Appareil d'assistance respiratoire selon la revendication 1, **caractérisé en ce que** ladite enveloppe souple (12, 39) est réalisée dans un film plastique de quelques microns d'épaisseur.

3. Appareil d'assistance respiratoire selon l'une des revendications 1 ou 2, présentant la forme d'un masque (1, 20), dont ladite chambre (3) est délimitée par une coque creuse (2) destinée à être appliquée, par son ouverture (8) qui constitue ladite sortie de gaz, sur le visage (9) d'un patient en enfermant le nez (10) de celui-ci, l'entrée du gaz respiratoire (4) étant prévue dans le fond de ladite coque (2) et ledit organe d'appui présentant la forme d'un bourrelet (11) suivant le contour de l'ouverture (8) de ladite coque et interposé entre ladite ouverture et ledit visage du patient,
**caractérisé en ce que** :
- ladite enveloppe souple est un manchon (12) replié autour dudit bourrelet, ledit manchon comportant une partie interne (12I) à ladite coque et une partie externe (12E) à ladite coque, reliées l'une à l'autre par une partie intermédiaire (12i) recourbée autour dudit bourrelet (11) ;
- ladite partie interne (12I) de l'enveloppe souple (12) définit avec la paroi interne (2I) de ladite coque (2) un espace interne (14) ;
- ladite partie externe (12E) de l'enveloppe souple (12) définit avec la paroi externe (2E) de ladite coque (2) un espace externe (15) ; et
- ledit espace interne (14) est en communication avec ladite chambre (3), tandis que ledit espace externe (15) est en communication avec l'extérieur (13).

4. Appareil d'assistance respiratoire selon la revendication 3,
**caractérisé en ce que** ledit manchon (12) est rapporté de façon amovible à ladite coque (2).

5. Appareil d'assistance respiratoire selon la revendication 3,
**caractérisé en ce que** ledit manchon (12) est solidaire de ladite coque (2).

6. Appareil d'assistance respiratoire selon la revendication 3,
**caractérisé en ce que** ledit espace externe (15) est ouvert vers l'extérieur (13).

7. Appareil d'assistance respiratoire selon la revendication 3,
**caractérisé en ce que** ledit espace externe (15) est en communication avec l'extérieur (13) grâce à au moins un orifice (23) pratiqué à travers ladite partie externe (12E) du manchon (12).

8. Appareil d'assistance respiratoire selon l'une des revendications 1 ou 2, comportant un élément tubulaire (31), dont le volume interne délimite ladite chambre (32) et qui est destiné à être introduit dans une narine (33) du patient, lesdites entrée et sortie de gaz respiratoire étant formées par les extrémités (35, 36) opposées dudit élément tubulaire (31) et ledit organe d'appui se présentant sous la forme d'un ballonnet (37) porté par la paroi externe (31E) dudit élément tubulaire (31) et interposé entre celui-ci et la paroi interne (33I) de ladite narine (33),
**caractérisé en ce que** ladite enveloppe souple présente la forme d'un ballon (39) enveloppant ledit ballonnet (37) et porté par ladite paroi externe (31E) dudit élément tubulaire (31) et **en ce que** ledit ballon (39) est en communication, à son extrémité distale, avec ladite chambre (32) par l'intermédiaire d'au moins un orifice (40) pratiqué à travers la paroi dudit élément tubulaire (31) et, à son extrémité proximale, avec l'extérieur (41), par l'intermédiaire d'au moins un orifice (42) pratiqué à travers la paroi dudit ballon (39).

## Patentansprüche

1. Beatmungsvorrichtung (1, 20, 30), die es ermöglicht, den Lungen eines Patienten Atemgas aus einer äußeren Quelle zuzuführen, wobei die genannte Vorrichtung Folgendes umfasst:
- eine Kammer (3, 32), die mit einem Atemgaseingang (4, 35), der mit der Quelle verbunden werden soll, und einem Atemgasausgang (8, 36), der wiederum mit einem Atemweg (10, 33) des Patienten verbunden werden soll, versehen ist; und
- ein Auflageorgan (11, 37), das auf einen Teil (9, 331) des Patienten aufgelegt werden soll, um die Dichtheit zwischen dem Atemgasausgang und der Außenumgebung zu gewährleisten,
**dadurch gekennzeichnet,**
- **dass** sie eine biegsame Hülle (12, 39) umfasst, die das Auflageorgan (11, 37) umschließt;
- **dass** die Biegsamkeit der Hülle (12, 39) größer ist als die des Auflageorgans (11, 37); und
- **dass** der biegsamen Hülle von einer Seite des Auflageorgans (11, 37) Atemgas zugeführt wird und sie von der anderen Seite des Auflageorgans mit der Außenumgebung (13, 41 verbunden ist, um das Atemgas abzuführen, sodass, wenn das Auflageorgan (11, 37) nicht dicht auf dem Teil des Patienten aufliegen kann, der dadurch entstehende Raum durch die aufgeblasene biegsame Hülle (12, 39) verschlossen wird.

2. Beatmungsvorrichtung nach Anspruch 1, **dadurch gekennzeichnet, dass** die biegsame Hülle (12, 39) aus einer einige Mikrometer starken Kunststofffolie hergestellt ist.

3. Beatmungsvorrichtung nach Anspruch 1 oder 2, die die Form einer Maske (1, 20) besitzt, deren Kammer (3) von einer hohlen Schale (2) begrenzt wird, die mit ihrer Öffnung (8), die den Gasausgang bildet, auf das Gesicht (9) eines Patienten aufgelegt werden soll, indem sie dessen Nase (10) umschließt, wobei der Atemgaseingang (4) im Boden der Schale (2) vorgesehen ist und das Auflageorgan die Form einer Wulst (11) entlang der Kontur der Öffnung (8) der Schale besitzt und sich zwischen der Öffnung und dem Gesicht des Patienten befindet,
**dadurch gekennzeichnet, dass**
- die biegsame Hülle eine Manschette (12) ist, die um die Wulst geklappt ist, wobei die Manschette einen in Bezug auf die Schale inneren Teil (121) und einen in Bezug auf die Schale äußeren Teil (12E) umfasst, die durch einen um die Wulst (11) gebogenen Zwischenteil (12i) miteinander verbunden sind;
- der Innenteil (121) der biegsamen Hülle (12) mit der Innenwand (21) der Schale (2) einen Innenraum (14) bildet;
- der Außenteil (12E) der biegsamen Hülle (12) mit der Außenwand (2E) der Schale (2) einen Außenraum (15) bildet; und
- der Innenraum (14) mit der Kammer (3) verbunden ist, wohingegen der Außenraum (15) mit der Außenumgebung (13) verbunden ist.

4. Beatmungsvorrichtung nach Anspruch 3,
**dadurch gekennzeichnet, dass**
die Manschette (12) abnehmbar an die Schale (2) angebaut ist.

5. Beatmungsvorrichtung nach Anspruch 3,
**dadurch gekennzeichnet, dass**
die Manschette (12) fest mit der Schale (2) verbunden ist.

6. Beatmungsvorrichtung nach Anspruch 3,
**dadurch gekennzeichnet, dass**
der Außenraum (15) nach außen (13) hin offen ist.

7. Beatmungsvorrichtung nach Anspruch 3,
**dadurch gekennzeichnet, dass**
der Außenraum (15) über mindestens eine durch den Außenteil (12E) der Manschette (12) führende Öffnung (23) mit der Außenumgebung (13) verbunden ist.

8. Beatmungsvorrichtung nach Anspruch 1 oder 2,
die ein rohrförmiges Element (31) umfasst, dessen Innenvolumen die Kammer (32) begrenzt und das in ein Nasenloch (33) des Patienten eingeführt werden soll, wobei der Atemgaseingang und -ausgang durch die gegenüberliegenden Enden (35, 36) des rohrförmigen Elements (31) gebildet werden und das Auflageorgan die Form eines kleinen Ballons (37) hat, das von der Außenwand (31E) des rohrförmigen Elements (31) getragen wird und zwischen diesem und der Innenwand (331) des Nasenlochs (33) liegt,
**dadurch gekennzeichnet, dass**
die biegsame Hülle die Form eines Ballons (39) besitzt, der den kleinen Ballon (37) umhüllt und von der Außenwand (31E) des rohrförmigen Elements (31) getragen wird, und dadurch, dass der Ballon (39) an seinem entfernten Ende über mindestens eine durch die Wand des rohrförmigen Elements (31) führende Öffnung (40) mit der Kammer (32) und an seinem nahen Ende über mindestens eine durch die Wand des Ballons (39) führende Öffnung (42) mit der Außenumgebung (41) verbunden ist.

## Claims

1. Respiratory assistance apparatus (1, 20, 30) making it possible for a respiratory gas coming from an external source to be taken into the lungs of a patient, said apparatus comprising:
- a chamber (3, 32) provided with a respiratory-gas inlet (4, 35) intended to be connected to said source and with a respiratory-gas outlet (8, 36) intended to be connected to an airway (10, 33) of said patient; and
- a support member (11, 37) intended to bear on a part (9, 33I) of said patient in order to seal between said respiratory-gas outlet and the outside,
**characterized:**
- **in that** it includes a flexible envelope (12, 39) surrounding said support member (11, 37);
- **in that** the flexibility of said envelope (12, 39) is greater than that of said support member (11, 37); and
- **in that** said flexible envelope, at one end of said support member (11, 37), is supplied with respiratory gas, and, at the other end of said support member, communicates with the outside (13, 41) in order to evacuate said respiratory gas so that, when said support member (11, 37) cannot be sealingly applied against said part of the patient, the space which results therefrom is closed off by said inflated flexible envelope (12, 39).

2. Respiratory assistance apparatus according to Claim 1,
**characterized in that** said flexible envelope (12, 39) is made of a plastic film a few microns in thickness.

3. Respiratory assistance apparatus according to either of Claims 1 or 2, having the shape of a mask (1, 20), said chamber (3) of which is bounded by a hollow shell (2) intended to be applied, by its opening (8) which constitutes said gas outlet, to the face (9) of a patient, enclosing the latter's nose (10), the respiratory-gas inlet (4) being provided in the bottom of said shell (2) and said support member having the shape of a bead (11) going around the perimeter of the opening (8) of said shell and interposed between said opening and said face of the patient,
**characterized in that**:
- said flexible envelope is a sleeve (12) folded back around said bead, said sleeve having a part (12I) internal to said shell and a part (12E) external to said shell, these parts being connected to each other by an intermediate part (12i) bent back around said bead (11);
- said internal part (12I) of the flexible envelope (12) defines with the internal wall (2I) of said shell (2) an internal space (14); and
- said external part (12E) of the flexible envelope (12) defines with the external wall (2E) of said shell (2) an external space (15); and
- said internal space (14) communicates with said chamber (3) while said external space (15) communicates with the outside (13).

4. Respiratory assistance apparatus according to Claim 3,
**characterized in that** said sleeve (12) is removably attached to said shell (2).

5. Respiratory assistance apparatus according to Claim 3,
**characterized in that** said sleeve (12) is fastened to said shell (2).

6. Respiratory assistance apparatus according to Claim 3,
**characterized in that** said external space (15) is open to the outside (13).

7. Respiratory assistance apparatus according to Claim 3,
**characterized in that** said external space (15) communicates with the outside (13) by means of at least one orifice (23) provided through said external part (12E) of the sleeve (12).

8. Respiratory assistance apparatus according to either of Claims 1 or 2, including a tubular element (31), the internal volume of which defines said chamber (32) and which is intended to be inserted into a nostril (33) of the patient, said respiratory-gas inlet and outlet being formed by the opposite ends (35, 36) of said tubular element (31) and said support member being in the form of a small balloon (37) supported by the external wall (31E) of said tubular element (31) and interposed between the latter and the internal wall (33I) of said nostril (33),
**characterized in that** said flexible envelope is in the form of a balloon (39) surrounding said small balloon (37) and supported by said external wall (31E) of said tubular element (31) and **in that** said balloon (39) communicates, at its distal end, with said chamber (32) via at least one orifice (40) provided through the wall of said tubular element (31), and, at its proximal end, with the outside (41) via at least one orifice (42) provided through the wall of said balloon (39).
